# EUROPÄISCHE PATENTSCHRIFT

(11) **EP 0 998 212 B1**
(45) Veröffentlichungstag und Bekanntmachung des Hinweises auf die Patenterteilung: **17.04.2002**
(21) Anmeldenummer: 98934773.7
(22) Anmeldetag: 20.05.1998
(51) Int. Cl.: A61B 1/12

(54) **LUFT-, WASSER- UND ABSAUGVENTILE AN ENDOSKOPEN**
WATER, AIR AND SUCTION VALVES ON ENDOSCOPES
SOUPAPES A AIR ET A EAU ET SOUPAPES D'ASPIRATION MONTEES DANS DES ENDOSCOPES

(30) Priorität: 24.07.1997 DE 19731965
(43) Veröffentlichungstag der Anmeldung: 10.05.2000
(73) Patentinhaber: XION GmbH, 13127 Berlin (DE)
(72) Erfinder: WIMMER, Viktor, Josef, D-80807 München (DE)
(74) Vertreter: Eder, Christian, Dipl.-Ing.
(86) Internationale Anmeldenummer: DE9801408
(87) Internationale Veröffentlichungsnummer: WO9904682

(56) Entgegenhaltungen:
- WO-A-94/19030
- US-A- 5 348 555

## Beschreibung

Die Erfindung betrifft ein Endoskop, insbesondere flexibles Endoskop, mit den Merkmalen des Oberbegriffs des Anspruchs 1.

Solche Endoskope weisen üblicherweise zwei Ventile zur Steuerung von Kanälen, wie beispielsweise Luftkanal, Wasserkanal und Absaugkanal, welche im Inneren des Endoskopgehäuses vom distalen bis zum proximalen Ende verlaufen, auf.

Durch diese Kanäle und deren Ventile können vom Bedienpersonal, beispielsweise ein Arzt, verschiedenste Aufgaben erledigt werden. Beispielsweise kann durch eine Betätigung des Luft-Wasserventils eine am distalen Ende des Endoskops befindliche verschmutzte Optik mit Wasser oder einem Luft-Wassergemisch gespült werden. Andererseits dient eine andere Betätigungsstufe des Luft/Wasserventils dazu, innere Organe wie z.B. Darm, Magen usw., zu Beobachtungs- oder Operationszwecken oder für die weiterführende Endoskopbewegung aufzublasen.

Durch eine Betätigung des Absaugventils können dagegen vom distalen Ende Schmutz, Sekrete, Wasser, Blut oder andere Flüssigkeiten, Luft usw. über den Absaugkanal abgesaugt werden.

Die Kanäle sind hierfür am proximalen Ende mit geeigneten Quellen, also Luftüberdruck, Wassertank und Luftunterdruck bzw. Vakuum angeschlossen.

Insbesondere der Absaugkanal unterliegt hierbei einer möglichen Verschmutzung durch verschiedene Sekrete oder auch durch kleine Feststoffe wie Körner o.ä..

Zum Reinigen können deshalb bei üblichen Endoskopen die Ventilkolben aus den Zylindern, beispielsweise durch ein Abschrauben der Manschetten, herausgezogen und abgenommen werden. Die Ventilkolben sind hierdurch gut extern reinigbar, wogegen die Ventilzylinder im Endoskopgehäuse mit Bürsten gereinigt werden müssen. Da die Ventilöffnungen in den Ventilzylindern nur schwer zugänglich sind, kann eine hygienisch einwandfreie Reinigung dieser Öffnungen und der sich daran anschließenden Kanäle nur mit größerem Aufwand bewerkstelligt werden.

Der vorliegenden Erfindung liegt daher die Aufgabe zugrunde, ein Ventil für ein Endoskop, sowie ein Endoskop mit am proximalen Ende des Endoskops angeordneten Ventilen zu schaffen, das eine hygienisch einwandfreie Reinigung der Ventile und der sich daran anschließenden Kanalbereiche auf einfache und bequeme Weise, schnell und kostengünstig ermöglicht.

Diese Aufgabe wird erfindungsgemäß mit den Merkmalen der Ansprüche 1 und 7 gelöst.

Durch die Ausbildung mehrerer Ventile als ein Ventilmodul, welches mit einer dazu komplementären Ausnehmung des Endoskopgehäuses lösbar verbunden ist, kann nicht nur wie bei herkömmlichen Endoskopen der Ventilkolben zu Reinigungszwecken aus dem Ventilzylinder herausgenommen werden, sondern auch die Ventilzylinder selbst. Hierdurch kann bei dem erfindungsgemäßen Endoskop nun der gesamte, mehrere Ventile umfassende Ventilblock bzw. das Ventilmodul vom Bedienpersonal heraus- bzw. abgenommen werden und vorteilhafterweise leicht zugänglich, beispielsweise in einem Autoklav, hygienisch einwandfrei gereinigt werden.

Zudem erleichtert sich durch die notwendigerweise gegenüber einem Ventilzylinder größer ausgebildete Ausnehmung im Endoskop und der daraus resultierenden besseren Zugänglichkeit eine Reinigung mittels Bürsten.

Die erfindungsgemäße Ausbildung erleichtert zudem durch die verbesserte Zugänglichkeit von Ventil bzw. Ventilmodul und Ausnehmung im Endoskop Wartungs- und Reparaturarbeiten. Beispielsweise können alte oder andersgeartete Ventile durch einfaches Ersetzen des gesamten Moduls auf einfache Weise auch durch das Bedienpersonal durch neue oder Spezialventile für einen bestimmten Anwendungszweck gewechselt bzw. ausgetauscht werden.

In einer Ausführungsform der Erfindung ist der Ventilkolben des wenigstens einen Ventils in dem Ventilzylinder herausnehmbar angeordnet. Hierdurch erhöhen sich die Reinigungs-, Reparatur- und Wartungsmöglichkeiten weiter, da jetzt zusätzlich auch der Innenraum eines jeden Ventils durch das Herausnehmen des Ventilkolbens leicht zugänglich ist, und Innenraum sowie Kolben auf einfache und komfortable Weise, beispielsweise in einem Autoklav, hygienisch einwandfrei gereinigt werden können.

In der bevorzugten Ausführungsform der Erfindung sind die Ventile als Luft/Wasser-Ventil und/oder Absaugventil ausgebildet. Hierdurch stehen einer Bedienperson die Möglichkeiten offen:
1. Luft vom proximalen Ende bzw. von einer an dem Versorgungsschlauch angeschlossenen Überdruckquelle über das Luft/Wasserventil ins distale Ende in ein Organ, wie beispielsweise Magen, Darm usw. der zu behandelnden Person zu blasen. Ein derartiges Aufblasen ist oft für eine bessere Beweglichkeit des Endoskopkopfes beim Einführen oder für eine Operation am Zielort notwendig.
2. Durch einen Stoß mit Wasser oder Luft-Wasser-Gemisch ist es der Bedienperson ebenfalls durch eine andere Betätigungsstufe des Luft/Wasserventils möglich, am distalen Ende verschmutzte Instrumente wie beispielsweise die Optik zu spülen, ohne die Behandlung durch ein Herausziehen des Endoskops zu Unterbrechen.
3. Schließlich können vom distalen Ende durch Betätigung des Absaugventils Sekrete, Feststoffe, Blut, Wasser und andere Flüssigkeiten aus medizinischen Gründen oder für medizinische Zwecke abgesaugt werden.

Bei der bevorzugten Ausführungsform der Erfindung ist deshalb das Luft/Wasserventil sowohl mit den im Inneren eines Versorgungsschlauchs liegenden Luft- und Wasserkanälen als auch mit den im Gehäuse des Endoskops in Richtung des Endoskopschafts führenden Luft- und Wasserkanälen verbunden. Durch verschiedene Kolbenstellungen können dann die unterschiedlichen Stufen - 0-Stellung, Luft, Luft-Wasser-Gemisch und nur Wasser - leicht von einer Bedienperson gesteuert werden.

In einer weiteren Ausführungsform der Erfindung ist das Ventilmodul in die Ausnehmung mittels einer Führung einschiebbar und fixierbar. Hierdurch erleichtert sich das Einsetzen und das Herausnehmen des Ventilmoduls, da wegen der hierdurch erfolgenden automatischen Selbstzentrierung des Ventilmoduls auf eine exakte Endposition nicht mehr geachtet werden muß.

Hierbei kann die Führung einen horizontalen und/oder vertikalen Vorschub ermöglichen, so dass beim Einsetzen des Ventilmoduls in die Ausnehmung alle Dichtstellen gleichzeitig und gleichmäßig in ihre entsprechenden Kanaleingänge an- und/oder eingepresst werden.

Durch die separate Ausbildung eines Ventilmoduls nach der Erfindung ist es auch möglich, dieses als Ersatzteil einzeln bereitzustellen, so dass eine sonst teure Reparatur durch einfaches Austauschen vermieden werden kann.

Weitere Ausführungsformen der Erfindung ergeben sich aus den Unteransprüchen.

Die Erfindung wird nachfolgend anhand eines in der Zeichnungen dargestellten Ausführungsbeispiels näher erläutert. In der Zeichnung zeigen:
- Fig. 1: eine Seitenansicht eines Endoskops mit Ventilmodul in eingesetztem Zustand und
- Fig. 2: eine teilweise aufgebrochene Seitenansicht eines Endoskops nach Fig. 1 in Explosionsdarstellung.

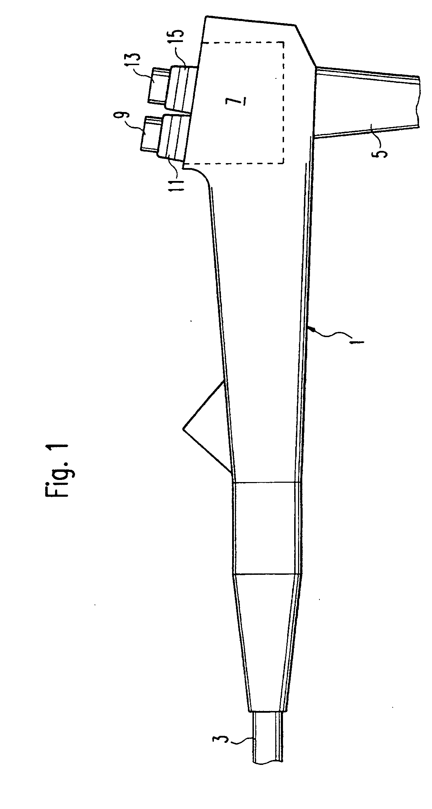

Das in Fig. 1 dargestellte Endoskop 1 umfasst ein Endoskopgehäuse 1 und einen sich daran anschließenden flexiblen, bewegbaren Endoskopeinführschaft 3, an dessen distalem Ende ein in der Zeichnung nicht näher dargestellter Endoskopkopf angeordnet ist. An seinem proximalen Ende schließt sich an der Unterseite des Endoskopgehäuses einen Versorgungsschlauch 5 an, welcher zu in der Zeichnung nicht näher dargestellten Luftüberdruck-, Luftunterdruck- und Wasserquellen führt.

An seiner dem Versorgungsschlauch gegenüberliegenden Seite ist in das proximale Gehäuseende 1 des Endoskops ein Ventilmodul 7 eingesetzt, welches ein vorderes Luft/Wasserventil mit Kolben 9 und ein hinteres Absaugventil mit Kolben 13 umfasst. Wie aus der Zeichnung ersichtlich, ragen die Ventile mit ihren oberen Bereichen zum manuellen Betätigen über ihre jeweiligen Verschlussmanschetten 11, 15 hinaus. In den Manschetten 11, 15 ist üblicherweise jeweils eine Druckfeder angeordnet, durch welche sich der jeweilige obere Bereich der Ventilkolben 9, 13 gegen das ortsfeste Gehäuse bzw. Manschette abstützt und mit einer nach oben gerichteten Federkraft beaufschlagt wird.

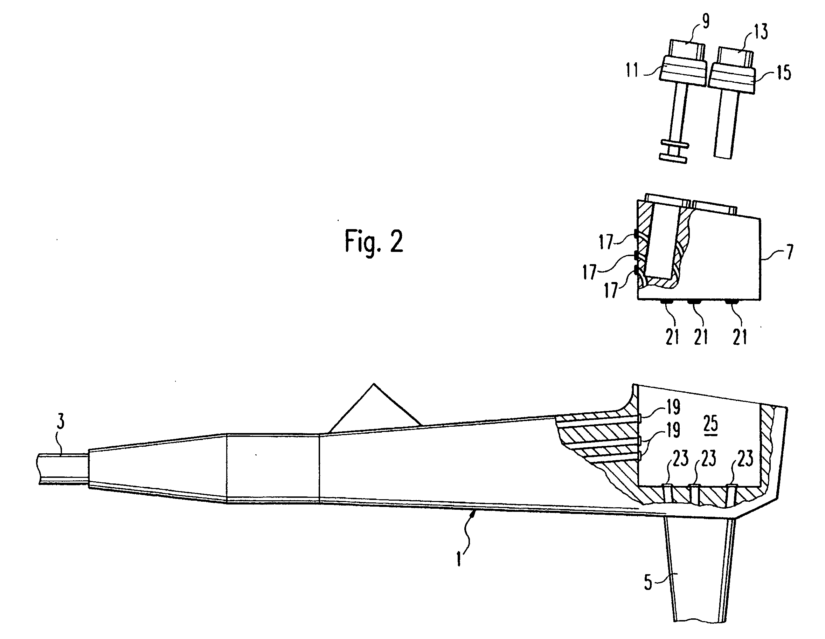

Wie in Fig. 2 dargestellt, weist das Ventilmodul eine quaderförmige Gestalt auf, in dessen Innerem nach oben offene Ventilzylinder ausgebildet sind. In diese Ventilzylinder können die Ventilkolben 9, 13 eingesetzt werden, wobei die Verschlussmanschetten 11, 15 in nicht näher dargestellter Weise mit ihrer Unterseite über einen nach oben ragenden Stutzen mit außenliegender Ringwulst gestülpt werden. Selbstverständlich sind für die Fixierung der Ventilkolben 9, 13 in ihren jeweiligen Zylindern auch andere Befestigungsmöglichkeiten, wie z.B. ein Schraub- oder Steckverschluss, denkbar.

In eingesetztem Zustand befindet sich das Modul 7 in einer hierzu komplementären Ausnehmung 25 am proximalen Ende des Endoskopgehäuses 1. Das Ventilmodul 7 weist an seiner in eingesetztem Zustand mit dem Gehäuse 1 in Eingriff kommenden Vorderseite drei vordere Dichtstellen 17 auf, die in eingesetztem Zustand in der Ausnehmung 25 mit den Kanaleingängen 19 zum Schaft 3 in Verbindung stehen. An seiner Unterseite weist das Ventilmodul 7 drei untere Dichtstellen 21 auf, die in eingesetztem Zustand in der Ausnehmung 25 mit den Kanaleingängen 23 zum Versorgungsschlauch 5 in Verbindung stehen. Wie in der Zeichnung nur teilweise dargestellt, sind die unteren Dichtstellen 21 über Kanäle oder Leitungen im Inneren des Ventilmoduls mit den jeweiligen Öffnungen in den Ventilzylindern verbunden. In ähnlicher Weise führen von den vorderen Dichtstellen 17 Leitungen oder Kanäle ins Innere des Ventilmoduls 7 zu ihren korrespondierenden Öffnungen der Ventilzylinder.

Im seinem, wie in Fig. 1 dargestellten, im Endoskop eingesetzten Zustand verbindet das Ventilmodul 7 die im Versorgungsschlauch liegenden, Luftüberdruck, Luftunterdruck und Wasser (unter Druck stehend) führenden Kanäle mit den im Gehäuseinneren liegenden, in Richtung Schaft 3 bis zum distalen Endoskopkopf führenden Kanälen. Selbstverständlich sind diese Kanäle gegen das Innere des Endoskopgehäuses 1 ebenso wie die Kanäle oder Leitungen des Ventilmoduls 7 gegen das Innere des Moduls 7 abgedichtet.

Durch das Absaugventil können durch dessen Betätigung, also Drücken von außen, die üblicherweise im Zylinder seitlich angeordneten Öffnungen geöffnet oder verschlossen werden, so dass je nach Betätigung eine Verbindung zwischen korrespondierenden Öffnungen entsteht bzw. unterbrochen wird. Hierdurch kann ein Arzt beispielsweise bei Bedarf zu einer am distalen Ende angeordneten Düse ein am proximalen Ende anliegendes Vakuum bzw. einen Unterdruck durchstellen und hierdurch Flüssigkeiten und/oder Feststoffe absaugen.

Im Zylinder des Luft/Wasserventils münden jeweils zwei zuführende und abgehende Kanäle, so dass mit diesem Ventil zwischen mehreren Zuständen zur Steuerung von distalen Funktionen gewählt werden kann. Wird nur der Luftunterdruckkanal durchverbunden, so entsteht an einer am distalen Ende angeordneten Düse ein Luftstrahl, der beispielsweise ein Aufblasen eines inneren Organs wie Magen, Darm o.ä. gestattet. Wird dagegen in gleicher Weise der Wasserkanal durchverbunden, so entsteht an einer am distalen Ende angeordneten Düse ein Wasserstrahl, welcher beispielsweise der Spülung einer etwaig verschmutzten Optik dienen kann. In einer Zwischenstufe können dabei auch Luft- und Wasserkanal durchverbunden sein, so dass am distalen Ende ein Luft/Wasser-Gemisch entsteht, welches im Bedarfsfall ebenfalls der Spülung der distalen Optik dient.

Das Sperren und das Durchverbinden der Kanäle wird hierbei jeweils durch eine Positionsveränderung des jeweiligen Ventilkolbens 9, 13 in seiner Längsrichtung bewerkstelligt. Hierbei verschließt bzw. öffnet der Ventilkolben 9, 13 mit an seinem Außenumfang befindlichen Vorsprüngen, beispielsweise in Form von O-Ringen o.ä. Dichtungen, die Öffnungen im Ventilzylinder.

Obwohl das in der Zeichnung dargestellte Ventilmodul 7 eine quaderförmige Form aufweist, sind unabhängig von diesem Ausführungsbeispiel auch andere Außenabmessungen, wie beispielsweise konkav und/oder konvex gekrümmte Außenseiten und andere Grundrisse wie oval, mehreckig usw. denkbar.

Besonders vorteilhaft sind bei jeder Form eines Ventilmoduls an der Außenfläche des Ventilmoduls 7 angeordnete Führungsmittel, die mit entsprechenden an der Innenseite der Ausnehmung 25 angeordneten komplementären Führungsmitteln zusammenwirken.

Durch diese Führung kann beim Einschieben des Moduls 7 in die Ausnehmung 25 auch ein gleichzeitiger vertikaler und horizontaler Vorschub erreicht werden. Eine derartige schräge in einem Winkel zwischen der Achse der Kanäle im Versorgungsschlauch 5 und der Achse der in Richtung Schaft 3 führenden Kanäle liegende Führung gewährleistet ein einfaches und komfortables Einsetzen des Moduls 7 in die Ausnehmung 25. Hierdurch kann ein gleichzeitiges und gleichmäßiges Ein- bzw. Anpressen der Dichtstellen 17, 21 an ihren korrespondierenden Kanaleingängen 19, 23 erreicht werden.

Vorteilhafterweise sind die Dichtstellen 17, 21 mit Dichtmitteln, beispielsweise Dichtungsringen, Gummistutzen o. ä, ausgestaltet, die in eingesetztem Zustand mit den evtl. konzentrisch erweiterten Kanaleingängen 19, 23 zusammenwirken. In diesem Fall können die nicht verschleißfreien Teile einer dichtenden Verbindung, nämlich die Dichtungsringe, auf einfache Weise an dem leicht zugänglichen Ventilmodul ersetzt werden. Es ist aber auch die kinematische Umkehr der Anordnung oder die Ausstattung von Dichtstellen 17, 21 und Kanaleingängen 19, 23 mit Dichtmitteln denkbar.

Unabhängig von der speziellen Ausführungsform kann die dichtende Verbindung zusätzlich durch herausragende Rohrstutzen der Kanaleingänge 19, 23, welche in eingesetztem Zustand des Moduls 7 in die Dichtstellen 17, 21 eingreifen, unterstützt werden. Die Rohrstutzen können hierbei in Einschieberichtung abgeschrägt sein, um ein leichteres Eingreifen zu ermöglichen, ohne dass die Dichtungen in ihrer horizontalen Ebene kurz vor der Endposition zu sehr beansprucht werden. Hierdurch kann ein bei schräger Führung sonst erforderliches horizontales Spiel der Dichtungen verringert werden.

Besonders vorteilhaft ist, ebenfalls unabhängig von einer speziellen Ausführungsform der Erfindung, die durch Ausbildung eines Ventilmodul eröffnete Möglichkeit, die Kanaleingänge im Wesentlichen gerade in die Ausnehmung 25 einmünden zu lassen. Hierdurch kann zusätzlich zu der durch die Ausnehmung ermöglichte bereits beschriebenen leichteren Zugänglichkeit der Kanaleingänge auch eine leichtere Zugänglichkeit der Kanäle selbst gewährleistet werden. Bei einem üblichen Reinigen mit einer Bürste muss diese vom Bedienpersonal nun nicht mehr durch stark gekrümmte und deshalb schwierig zugängliche Kanaleingänge und -endbereiche geführt werden. Die Kanaleingänge liegen hierbei im Wesentlichen in Richtung ihrer gesamten jeweiligen Kanallängsachse und können schräg oder senkrecht in die Wände der Ausnehmung 25 einmünden.

Vorteilhafterweise können sie leicht schräg in Richtung der Öffnung der Ausnehmung 25 einmünden, um die Zugänglichkeit von außen für eine Reinigungsbürste weiter zu verbessern.

## Patentansprüche

1. Flexibles Endoskop,
mit wenigstens einem im proximalen Ende eines Endoskopgehäuses (1) angeordneten, einen Ventilkolben (9, 13) und einen Ventilzylinder mit mehreren Ventilöffnungen umfassenden Ventil,
**dadurch gekennzeichnet,**
**dass** das wenigstens eine Ventil als ein Ventilmodul (7) ausgebildet ist und
**dass** das Ventilmodul (7) mit einer dazu komplementären Ausnehmung (25) des Endoskopgehäuses (1) lösbar verbunden ist.

2. Endoskop nach Anspruch 1, **dadurch gekennzeichnet, dass** der Ventilkolben (9, 13) des wenigstens einen Ventils in dem Ventilzylinder herausnehmbar angeordnet ist.

3. Endoskop nach Anspruch 1 oder 2, **dadurch gekennzeichnet, dass** das Ventilmodul (7) an seiner mit der Ausnehmung (25) in Eingriff kommenden Außenfläche dichtende Anschlussstellen (17, 21) aufweist, die mit den Ventilöffnungen des wenigstens einen Ventilzylinders, gegen das Innere des Ventilmoduls (7) abgedichtet, verbunden sind und dass die Anschlussstellen (17, 21) des Ventilmoduls (7) in eingesetztem Zustand mit innerhalb des Endoskops ausgebildeten Eingängen (19, 23) der Kanäle, welche gegen das Innere des Endoskopgehäuses (1) abgedichtet sind, verbunden sind.

4. Endoskop nach einem der vorhergehenden Ansprüche, **dadurch gekennzeichnet, daß** das wenigstens eine Ventil als Luft-Wasser-Ventil oder Absaugventil ausgebildet ist, wobei die im Inneren eines Versorgungsschlauchs (5) liegenden Luftund Wasserkanäle mittels des Luft/Wasserventils mit im Gehäuse des Endoskops in Richtung des Endoskopschafts führenden Luft- und Wasserkanälen in unterschiedlicher Kombination verbindbar sind und/oder der im Inneren des Versorgungsschlauchs liegende Absaugkanal mittels des Absaugventils mit dem im Gehäuse des Endoskops in Richtung des Endoskopschafts führenden Absaugkanal verbindbar ist.

5. Endoskop nach einem der vorhergehenden Ansprüche, **dadurch gekennzeichnet, dass** das Ventilmodul (7) in die Ausnehmung mittels einer Führung einschiebbar und fixierbar ist.

6. Endoskop nach Anspruch 5, **dadurch gekennzeichnet, dass** die Führung als schräge Führung zwischen den Längsachsen der Kanäle in Schaftrichtung und der Kanäle des Versorgungsschlauchs ausgebildet ist, so dass alle Anschlussstellen (17, 21) beim Einschieben gleichzeitig und gleichmäßig in ihre entsprechenden Kanaleingänge (19, 23) an- und/oder eingepresst werden.

7. Ventilmodul für ein flexibles Endoskop, mit wenigstens einem einen Ventilkolben (9, 13) und einen Ventilzylinder mit mehreren Ventilöffnungen umfassenden Ventil, wobei das Ventilmodul (7) mit einer in einem Endoskopgehäuse (1) ausgebildeten, zu dem Ventilmodul (7) komplementären Ausnehmung (25) lösbar verbindbar ist.

8. Ventilmodul nach Anspruch 7, **dadurch gekennzeichnet, dass** der Ventilkolben (9, 13) in dem Ventilzylinder herausnehmbar angeordnet ist.

9. Ventilmodul nach Anspruch 7 oder 8, **dadurch gekennzeichnet, dass** das Ventilmodul an seiner in eingesetztem Zustand mit der Ausnehmung (25) in Eingriff kommenden Außenfläche dichtende Anschlussstellen (17, 21) aufweist, die mit den Ventilöffnungen des wenigstens einen Ventilzylinders, gegen das Innere des Ventilmoduls abgedichtet, verbunden sind und
dass die Anschlussstellen (17, 21) des Ventilmoduls (7) in eingesetztem Zustand mit innerhalb des Endoskops ausgebildeten Eingängen (19, 23) der Kanäle, welche gegen das Innere des Endoskopgehäuses (1) abgedichtet sind, verbunden sind.

## Claims

1. Flexible endoscope,
having at least one valve, which is disposed in the proximal end of an endoscope housing (1) and comprises a valve piston (9, 13) and a valve cylinder having a plurality of valve openings,
**characterized in**
**that** the at least one valve takes the form of a valve module (7) and
**that** the valve module (7) is detachably connected to a complementary recess (25) of the endoscope housing (1).

2. Endoscope according to claim 1, **characterized in that** the valve piston (9, 13) of the at least one valve is disposed in a removable manner in the valve cylinder.

3. Endoscope according to claim 1 or 2, **characterized in that** the valve module (7) at its outer surface coming into engagement with the recess (25) comprises sealing connection points (17, 21), which are connected to the valve openings of the at least one valve cylinder so as to be sealed off from the interior of the valve module (7) and
that the connection points (17, 21) of the valve module (7) in the inserted state are connected to inlets (19, 23), which are formed inside the endoscope, of the channels, which are sealed off from the interior of the endoscope housing (1).

4. Endoscope according to one of the preceding claims, **characterized in that** the at least one valve takes the form of an air-water valve or suction valve, wherein the air and water channels situated in the interior of a flexible supply tube (5) are connectable by means of the air-water valve in differing combination to air and water channels leading in the housing of the endoscope towards the endoscope stem and/or the suction channel situated in the interior of the flexible supply tube is connectable by means of the suction valve to the suction channel leading in the housing of the endoscope towards the endoscope stem.

5. Endoscope according to one of the preceding claims, **characterized in that** the valve module (7) is insertable into the recess by means of a guide and fixable.

6. Endoscope according to claim 6, **characterized in that** the guide takes the form of an oblique guide between the longitudinal axes of the channels in stem direction and of the channels of the flexible supply tube, so that all connection points (17, 21) during insertion are simultaneously and uniformly pressed onto or into their appropriate channel inlets (19, 23).

7. Valve module for a flexible endoscope, having at least one valve, which comprises a valve piston (9, 13) and a valve cylinder having a plurality of valve openings, wherein the valve module is detachably connectable to a recess (25), which is formed in an endoscope housing (1) and complementary to the valve module (7).

8. Valve module according to claim 7, **characterized in that** the valve piston (9, 13) is disposed in a removable manner in the valve cylinder.

9. Valve module according to claim 7 or 8, **characterized in that** the valve module at its outer surface coming in the inserted state into engagement with the recess (25) comprises sealing connection points (17, 21), which are connected to the valve openings of the at least one valve cylinder so as to be sealed off from the interior of the valve module and
that the connection points (17, 21) of the valve module (7) in the inserted state are connected to inlets (19, 23), which are formed inside the endoscope, of the channels, which are sealed off from the interior of the endoscope housing (1).

## Revendications

1. Endoscope flexible comportant au moins une soupape disposée dans l'extrémité proximale d'un boîtier d'endoscope (1) et comprenant un piston (9, 13) et un cylindre comportant plusieurs ouvertures de soupape,
**caractérisé en ce**
**que** la au moins une soupape est agencée sous la forme d'un module de soupape (7), et
**que** le module de soupape (7) est relié de façon amovible à un évidement complémentaire (25) du boîtier d'endoscope (1).

2. Endoscope selon la revendication 1, **caractérisé en ce que** le piston (9, 13) de la au moins une soupape est disposé dans le cylindre de la soupape de manière à pouvoir en être ressorti.

3. Endoscope selon la revendication 1 ou 2, **caractérisé en ce que** le module de soupape (7) comporte, au niveau de sa surface extérieure, qui vient coopérer avec l'évidement (25), des zones de raccordement étanches (17, 21), qui sont reliés aux ouvertures du au moins un cylindre de soupape, d'une manière étanche vis-à-vis de l'intérieur du module de soupape (7), et que les zones de raccordement (17, 21) du module (7) de soupape sont reliées, à l'état inséré, à des entrées (19, 23), formées à l'intérieur de l'endoscope, des canaux qui sont fermés de façon étanche vis-à-vis de l'intérieur du boîtier d'endoscope (1).

4. Endoscope selon l'une des revendications précédentes, **caractérisé en ce que** la au moins une soupape est agencée sous la forme d'une soupape à air/eau ou une soupape d'aspiration, les canaux de circulation d'air et d'eau, qui sont situés à l'intérieur d'un tuyau d'alimentation (5), pouvant être reliés selon une combinaison différente au moyen de la soupape à air/eau à des canaux pour l'air et l'eau, qui aboutissent dans le boîtier d'endoscope en direction de la tige de l'endoscope, et/ou le canal d'aspiration, qui est situé à l'intérieur du tuyau d'alimentation, peut être relié au moyen de la soupape d'aspiration au canal d'aspiration qui est ménagé dans le boîtier d'endoscope en direction de la tige de l'endoscope.

5. Endoscope selon l'une des revendications précédentes, **caractérisé en ce que** le module de soupape (7) peut être inséré et fixé au moyen d'un guide.

6. Endoscope selon la revendication 5, **caractérisé en ce que** le guide est agencé sous la forme d'un guide oblique entre les axes longitudinaux dans la direction de la tige et des canaux du tuyau d'alimentation de sorte que lors de l'insertion toutes les zones de raccordement (17, 21) sont serrées ou comprimées simultanément et uniformément dans leurs entrées correspondantes (19, 23) dans les canaux.

7. Module de soupape pour un endoscope flexible comportant au moins une soupape comportant un piston (9, 13) et un cylindre équipé de plusieurs ouvertures de soupape, le module de soupape pouvant être relié de façon amovible à un évidement (25) formé dans un boîtier (1) de l'endoscope et complémentaire du module de soupape (7).

8. Module de soupape selon la revendication 7, **caractérisé en ce que** le piston (9, 13) de la soupape est disposé, dans le cylindre de soupape, de manière à pouvoir en être ressorti.

9. Module de soupape selon la revendication 7 ou 8, **caractérisé en ce que** le module de soupape possède au niveau de sa surface extérieure qui, à l'état inséré, vient coopérer avec l'évidement (25) des zones de raccordement (17, 21) établissant l'étanchéité, qui sont reliées aux ouvertures du au moins un cylindre de soupape, d'une manière étanche vis-à-vis de l'intérieur du module de soupape, et
qu'à l'état inséré, les zones de raccordement (17, 21) du module de soupape (7) sont reliées à des entrées (19, 23), situées à l'intérieur de l'endoscope, des canaux qui sont fermés de façon étanche vis-à-vis de l'intérieur du boîtier (1) d'endoscope.
